# EUROPEAN PATENT APPLICATION

(11) **EP 1 748 038 A1**
(43) Date of publication of application: **31.01.2007**
(21) Application number: 05016525.7
(22) Date of filing: 29.07.2005
(51) Int. Cl.: C07C 2/30

(54) **Method for preparing linear alpha-olefins**

(71) Applicant: Linde AG, 65189 Wiesbaden (DE); Saudi Basic Industries Corporation, Riyadh 11422 (SA)
(72) Inventor: Fritz, Peter, Dr., 82008 Unterhaching (DE); Bölt, Heinz, 82515 Wolfratshausen (DE); Hoffmann, Karl-Heinz, Dr., 82110 Germering (DE); Köhler, Markus, 81543 München (DE); Mosa, Fuad, 11422 Riyadh (SA); Ali, Talal, Dr., P.O. Box 42503, Riyadh 11551 (SA); Zander, Hans-Jörg, Dr., 81479 München (DE)
(74) Representative: Winkler, Andreas Fritz Ernst

(57) **Abstract**

The present invention relates to a method for preparing linear alpha-olefins by oligomerizing of ethylene in the presence of an organic solvent and a homogenous catalyst, characterized in that the method is carried out in a reactor being fed with a gaseous feed comprising a minor amount of ethylene and a major amount of an inert gas.

## Description

The present invention relates to a method for preparing linear alpha-olefins by oligomerizing of ethylene in the presence of an organic solvent and a homogenous catalyst.

The oligomerization of ethylene using an organometallic catalyst is widely known in the art. The oligomerization is highly exothermic so that reaction heat has to be removed from the reactor to prevent a runaway.

DE 43 38 414 C1 discloses a method for the preparation of linear alpha-olefins, wherein polymer grade ethylene is re-circulated to remove the reaction heat. Therefore, ethylene feed (having an ethylene content of approximately 100% with minor amounts of impurities) is introduced into the reactor at a lower temperature, and non-oligomerized monomeric ethylene is removed at a higher temperature, cooled down and re-introduced into the reactor.

It was found that only about 3% of the ethylene feed is used in the oligomerization process, wherein the remainder is utilized as cooling media. Ethylene is quite expensive.

Thus, it is an object of the present invention to provide a method for preparing linear alpha-olefins by oligomerizing of ethylene, which method overcomes the disadvantages of the prior art. Especially, a method shall be provided which may be carried out in a more cost-effective manner.

This object is achieved in that the method is carried out in a reactor being fed with a gaseous feed comprising a minor amount of ethylene and a major amount of an inert gas.

Preferably, the feed comprises at least about 3 wt.% of ethylene.

Most preferably, the feed comprises about 5 to about 10 wt.% of ethylene.

In one embodiment, the inert gas is selected from any of the rare gases, methane, ethane, hydrogen, nitrogen SF₆, propane, propylene, butane or mixtures thereof, wherein ethane is preferred.

The organic solvent may be toluene.

Preferably, the catalyst comprises a zirconium salt of organic acids and at least one organoaluminum compound.

It is proposed that the zirconium salt has the formula ZrCl₄₋ₘXₘ, wherein X = OCOR or OSO₃R' with R and R' being independently alkyl, alkene or phenyl, and wherein 0 < m < 4.

The at least one aluminum compound may be Al(C₂H₅)₃, Al₂Cl₃(C₂H₅)₃, AlCl(C₂H₅)₂ or mixtures thereof.

The oligomerization may be carried out in the reactor at a temperature between about 60 to about 100°C.

Finally, the feed may be introduced into the reactor at a temperature of about 20 to about 50°C.

Surprisingly, it was found that instead of polymer grade ethylene which has been used both so far as starting material and cooling media, this monomer can be substantially replaced by an inert gas only providing the cooling function, but not taking part in the oligomerization process.

Utilizing the inventive method, a significant reduction of the ethane/ethylene purge stream from the ethylene cycle (in the amount from t/h to kg/h) is achievable, which may be routed back for reprocessing to an ethylene plant or even has to be considered as a loss. Any purge gas from a polyethylene plant may be utilized as feedstock for the LAO plant utilizing the inventive method.

Any inert gas which does not liquefy under the oligomerization conditions may be utilized. The preferred inert gas is ethane. Utilizing ethane, the application of low-cost feedstock is possible, since the feedstock can be easily withdrawn upstreams of the high energy-consuming C₂-splitter of an ethylene plant and may be fed directly into the reactor for oligomerizing of ethylene. However, any other suitable inert gas may be chosen.

It was found that the partly replacement of the conventional 100% ethylene feed by an inert gas does not influence the oligomerization rate and any process conditions.

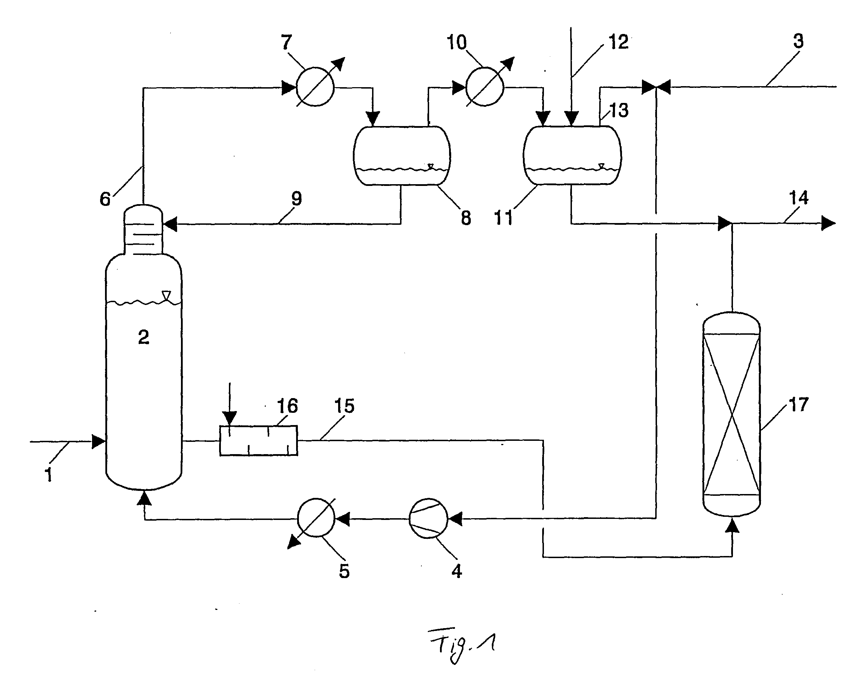

The invention is now further illustrated with reference to the accompanying drawing wherein figure 1 schematically illustrates an inventive method for preparing of linear alpha-olefins by oligomerizing of ethylene.

In the plant illustrated in figure 1 catalyst dissolved in toluene is fed to oligomerization reactor 2 via line 1. Via line 3 a feed comprising ethylene and an inert gas, e.g. ethane, is provided. Preferably, the feed comprises about 5 to about 10 wt.% of ethylene. The feed is re-circulated through the reactor, two cooling devices, two separators and a compressor and a heater, to remove the reaction heat from the reactor 2. The feed is compressed in the compressor 4, heated in the trim heater 5 to a temperature of about 20°C and is introduced into the oligomerization reactor 2 from the bottom. In the reactor 2 the oligomerization of ethylene is conducted, when the feed bubbles through the mixture of solvent and catalyst. The products of the oligomerization remain dissolved in the solvent. The temperature in the reactor is about 60-100°C.

Via line 6 a mixture of ethylene and light alpha-olefins is removed, and, according to the thermodynamic equilibrium, some toluene. The mixture is cooled down in cooling device 7 to a temperature of about 35°C and is collected in separator 8. The liquid obtained, consisting of toluene and alpha-olefins, is re-circulated via line 9 into the separator part of the reactor 2. The part from the separator 8 remaining gaseous is further cooled in cooling device 10 to a temperature of about 5°C and is transferred into the separator 11. In the cooling device 10 the cooling is adjusted so that olefins heavier than ethylene are liquefied. Via line 12 the separator may be provided with a recycled C₂-fraction from a hydrocarbon separation plant (not shown). Via line 13 a mixture of non-consumed ethylene and inert gas may be mixed with a fresh feed thereof, wherein the admixture may be again introduced into the reactor 2 via line 3, compressor 4 and trim heater 5. The liquid products from the separator 11 are transferred via line 14 to an olefin separation (not shown), for example a rectification column.

Via line 15 a liquid mixture consisting of toluene, catalyst, dissolved ethylene and linear alpha-olefins, is removed and may be processed as is well known in the art utilizing a mixing unit 16 and an adsorber 17.

The features disclosed in the foregoing description, in the drawing or in the claims may, both separately and in any combination thereof, be material for realizing the invention in diverse forms thereof.

## Claims

1. Method for preparing linear alpha-olefins by oligomerizing of ethylene in the presence of an organic solvent and a homogenous catalyst, **characterized in that** the method is carried out in a reactor being fed with a gaseous feed comprising a minor amount of ethylene and a major amount of an inert gas.

2. Method according to claim 1, wherein the feed comprises at least about 3 wt.% of ethylene.

3. Method according to claim 2, wherein the feed comprises about 5 to about 10 wt.% of ethylene.

4. Method according to any of the preceding claims, wherein the inert gas is selected from any of the rare gases, methane, ethane, hydrogen, nitrogen SF₆, propane, propylene, butane or mixtures thereof, wherein ethane being preferred.

5. Method according to any of the preceding claims, wherein the organic solvent is toluene.

6. Method according to any of the preceding claims, wherein the catalyst comprises a zirconium salt of organic acids and at least one organoaluminum compound.

7. Method according to claim 6, wherein the zirconium salt has the formula ZrCl₄₋ₘXₘ, wherein X = OCOR or OSO₃R' with R and R' being independently alkyl, alkene or phenyl, and wherein 0 < m < 4.

8. Method according to claim 6, wherein the at least one aluminum compound is Al(C₂H₅)₃, Al₂Cl₃(C₂H₅)₃, AlCl(C₂H₅)₂ or mixtures thereof.

9. Method according to any of the preceding claims, wherein the oligomerization is carried out in the reactor at a temperature between about 60 to about 100°C.

10. Method according to any of the preceding claims, wherein the feed is introduced into the reactor at a temperature of about 20 to about 50°C.
